# EUROPÄISCHE PATENTSCHRIFT

(11) **EP 2 104 078 B1**
(45) Veröffentlichungstag und Bekanntmachung des Hinweises auf die Patenterteilung: **23.01.2013**
(21) Anmeldenummer: 08022116.1
(22) Anmeldetag: 19.12.2008
(51) Int. Cl.: G08B 29/14, G08B 17/10, G01N 29/11

(54) **Rauchwarnmelder**
Smoke alarm device
Détecteur de fumée

(30) Priorität: 19.03.2008 DE 102008015043
(43) Veröffentlichungstag der Anmeldung: 23.09.2009
(73) Patentinhaber: ista International GmbH, 45131 Essen (DE)
(72) Erfinder: Niederfeld, Gerhard, 45131 Essen (DE); Schnepel, Sebastian, 45257 Essen (DE)
(74) Vertreter: Cohausz Hannig Borkowski Wißgott

(56) Entgegenhaltungen:
- EP-A1- 1 857 989
- EP-A1- 1 870 866
- DE-U1- 20 108 451
- JP-A- 2 128 298

## Beschreibung

Die Erfindung betrifft ein Verfahren zur Feststellung der Verschmutzung der Raucheintritfsöffnungen eines Rauchwarnmelders mit einem elektroakustischen Schallgeber. Die Erfindung betrifft weiterhin einen Rauchmelder mit einer Messkammer, die durch Raucheintrittsöffnungen gegen die äußere Umgebung abgegrenzt ist, wobei an die Messkammer ein Schallgeber, insbesondere über einen Hohlkammerresonator, angekoppelt ist und der eine Elektronik zur Überprüfung der Verschmutzung der Raucheintrittsöffnungen aufweist.

Im Stand der Technik ist es bekannt, zur Auslösung eines Alarms bei vorhandenen Rauchgasen in Räumen von Gebäuden Rauchwarnmelder einzusetzen, die häufig auf einem optischen Messprinzip beruhen und durch eine optische Messung Partikel, wie beispielsweise Rauchpartikel, in der Luft des Gebäudes feststellen. Hierfür weisen derartige Rauchmelder eine Messkammer mit einer optischen Messstrecke auf, wobei die Messkammern zur Umgebung hin über Raucheintrittsöffnungen offen sind.

Derartige Raucheintrittsöffnungen, die z.B. durch eine Vielzahl von Löchern oder auch netzartig ausgebildet sein können, haben die Aufgabe, zum einen grobe Verschmutzungen in der Raumluft aus der Messkammer fernzuhalten ebenso wie Tiere, insbesondere Insekten und Spinnentiere, die gegebenenfalls ansonsten einen Weg in die Messkammer eines solchen Rauchmelders finden und zu einer Fehlalarmauslösung beitragen.

Darüber hinaus ist es bekannt, dass mit zunehmender Verschmutzung der Messkammer und insbesondere der Raucheintrittsöffnungen einer solchen Messkammer von Rauchmeldern die Ansprechempfindlichkeit derartiger Rauchmelder angehoben wird, was ebenso ab bestimmten Verschmutzungsgraden zu Fehlalarmen führen kann.

Ebenso kann es auch sein, dass durch eine starke Verschmutzung der Raucheintrittsöffnungen der Eintritt von Rauch aus der Luft eines Gebäudes bzw. Raumes in die Messkammer erschwert wird und hierdurch die Rauchmelder mit der Zeit zu unempfindlich werden und gegebenenfalls nicht mehr rechtzeitig auslösen. Dabei ist gerade bei der mit Feuer einhergehenden Rauchentwicklung ein möglichst frühzeitiges Auslösen eines Rauchmelders nötig, um die mit der Rauchentwicklung einhergehenden Vergiftungsgefahren zu reduzieren, die gegenüber den üblichen Gefahren durch Verbrennungen dominieren.

Im Stand der Technik ist es bereits bekannt, eingangs genannte Rauchmelder, die einen elektroakustischen Schallgeber aufweisen, elektronisch hinsichtlich des Grades der Verschmutzung zu überprüfen. Dabei wird von dem Prinzip Gebrauch gemacht, dass sich abhängig von der Verschmutzung die Resonanzfrequenz des elektroakustischen Schallgebers ändert. Durch eine Überprüfung der Resonanzfrequenz kann somit auch auf die Verschmutzung des Rauchmelders zurückgeschlossen werden.

JP 02128298 offenbart einen Rauchmelder mit einem Schallgeber für die Überpüfung der Verschmutzung.

Derartige Verfahren sind jedoch aufwendig und wenig sensitiv, da Verschiebungen in der Resonanzfrequenz lediglich geringfügiger Art sind und somit hochempfindliche Elektroniken bereitgestellt werden müssen, um derartige Verschiebungen in der Frequenz zuverlässig feststellen zu können.

Es ist daher die Aufgabe der Erfindung, ein Verfahren zur Feststellung der Verschmutzung der Raucheintrittsöffnungen eines Rauchmelders mit einem elektroakustischen Schallgeber bereitzustellen, ebenso wie einen Rauchmelder, der von einer elektronischen Überprüfung der Verschmutzung Gebrauch macht, wobei hier bevorzugt eine technisch wenig aufwendige, günstige und weiterhin zuverlässige Möglichkeit zur Feststellung der Verschmutzung geschaffen werden soll.

Die Aufgabe wird gemäß einem Aspekt der Erfindung durch ein Verfahren der eingangs genannten gattungsgemäßen Art gelöst, bei welchem der Schallgeber in einem ersten Schritt zur Erzeugung von Schall erregt wird. Diese Erregung kann insbesondere durch den in dem Rauchmelder ohnehin vorhandenen elektroakustischen Schallgeber erfolgen, der ansonsten zur Alarmauslösung eingesetzt wird. Natürlich kann auch ein separater Schallgeber eingesetzt werden.

Beispielsweise kann es sich bei der Anregung um eine transiente elektrische Anregung des elektroakustischen Schallgebers handeln. Bevorzugt wird eine Anregung nur über eine bestimmte vorgegebene bzw. vorgebbare Zeit durchgeführt, insbesondere also pulsartig ausgeführt, wobei sodann in einem zweiten nachfolgenden Schritt eine vom abklingenden Schall, bzw. der abklingenden Schwingung des Schallgebers oder einer daran angeordneten Signalelektrode abhängige elektrische Signal-Wechselspannung zumindest zeitweise gemessen wird, wobei aus den Messwerten, insbesondere aus dem Verlauf der Signal-Wechselspannung ein Maß für die Verschmutzung der Raucheintrittsöffnungen errechnet wird.

Gelöst wird die Aufgabe weiterhin durch einen Rauchmelder der eingangs genannten Art, der sich weiterhin dadurch auszeichnet, dass die Elektronik zur Überprüfung der Verschmutzung einen Schalter umfasst, mittels dem wahlweise ein Oszillator oder eine Messelektronik an den Schallgeber oder eine daran angeordnete Signalelektrode anschaltbar ist. Dabei ist bevorzugt die Messelektronik eingerichtet, die Beträge aufeinander folgender Extremwerte einer Signal-Wechselspannung zu erfassen, die am Schallgeber oder dessen Signalelektrode nach einer vom Oszillator erzeugten und mit dem Schalter abgeschalteten Anregungs-Wechselspannung anliegt und zeitlich abklingt. Unter aufeinanderfolgenden Extremwerten bzw. Spannungsextrema werden dabei bevorzugt aufeinanderfolgende gleichartige Extrema verstanden, also aufeinanderfolgende Spannungsmaxima bzw. aufeinanderfolgende Spannungsminima. Grundsätzlich ist es auch denkbar unmittelbar aufeinanderfolgende Extrema einer Signalspannung, also aufeinderfolgende Maxima und Minima zu erfassen und auszuwerten. Es können hierfür z.B. die Absolutbeträge der erfassten Messwerte gebildet und verwendet werden.

Der wesentliche Kerngedanke der Erfindung liegt somit darin, zur Erfassung einer Verschmutzung der Raucheintrittsöffnungen, beispielsweise einer Verstopfung derselben, die akustische Kopplung zwischen Umgebungsluft und Luft in der Messkammer zu erfassen. Hierbei wird durch die insbesondere pulsartige Anregung des elektroakustischen Schallgebers ein schwingendes System erzeugt, bei dem zwei schwingende Massen, nämlich die Umgebungsluft und die Luft in der Messkammer, über die Raucheintrittsöffnungen aneinander gekoppelt sind. Eine Verschmutzung dieser Raucheintrittsöffnungen wirkt sich somit als eine Drosselung dieser Kopplung aus und erhöht damit die Dämpfung von dem Schall, der durch die Messkammer austritt.

Anders als im eingangs zitierten Stand der Technik wird somit nicht von einer Verschiebung der Resonanzfrequenz Gebrauch gemacht, sondern davon, dass die Dämpfung, mit welcher ein pulsartig angestoßenes schwingendes System in seiner Schwingung abklingt, erfasst wird.

Dies hat insbesondere den Vorteil, dass die akustische Anregung für das menschliche Ohr kaum wahrnehmbar ist und somit die Überprüfung ohne Störung von evtl. anwesenden Personen durchgeführt werden kann. Darüber hinaus wird durch die pulsartige Anregung sichergestellt, dass ein für die Auswertung des Signalpegels der abklingenden Schwingung ausreichendes Maß an Signalenergie auf der Resonanzfrequenz des Rauchmelders, insbesondere des aus Schallgeber, Resonator, Messkammer und Umgebungsluft gebildeten schwingungsfähigen Systems bereitgestellt wird. Nach Abschalten der Anregung klingt die Bewegung der Luft, insbesondere im Wesentlichen auf der ersten Eigenfrequenz des Systems ab. Eine Veränderung des Abkling-Koeffizienten ist ein Maß für den Grad der Verschmutzung der Raucheintrittsöffnungen.

Für die Erzeugung des benötigten Schalls wird in einer bevorzugten Ausführung der Alarmgeber, insbesondere der elektroakustische Schallgeber, verwendet, der in einer bevorzugten Ausführung beispielsweise als Piezoschallgeber realisiert sein kann. Beispielsweise kann ein solcher Schallgeber als eine insbesondere runde Platte ausgebildet sein, vorzugsweise aus einem Metall, auf der eine piezoelektrische Scheibe, insbesondere eine runde Scheibe positioniert ist, die zu Schwingungen angeregt werden kann, beispielsweise über eine Querkontraktion zu einer Biegeschwingung, insbesondere der ersten Eigenform. Hierdurch kann in bevorzugter Ausführung ein Kolbenstrahler ausgebildet werden, der z. B. in einer möglichen Ausführung an einen Hohlkammerresonator angekoppelt ist. Dabei kann der Schallgeber zusammen mit dem Hohlkammerresonator so positioniert sein, dass der Schall durch die Messkammer geleitet wird und durch die Raucheintrittsöffnungen der Rauchkammer zur Umgebung geleitet wird.

Durch eine kurzzeitige, insbesondere impulsartige Anregung des Schallgebers wird das Gesamtsystem aus Schallgeber, Resonator, Messkammer bzw. Rauchkammer und der Umgebungsluft in Schwingungen versetzt,. Hierbei kann es vorgesehen sein, dass die elektrische Signal-Wechselspannung, die zumindest zeitweise gemessen wird, um auf die Dämpfung zu schließen, direkt am Schallgeber abgegriffen wird oder an einer mit diesem verbundenen Signalelektrode.

So wird demnach der eingangs genannte Schalter eines erfindungsgemäßen Rauchmelders eingesetzt, um zunächst die von einem Oszillator erzeugte elektrische Spannung an den Schallgeber anzuschalten und sodann mit dem Schalter den Oszillator vom Schallgeber abzuschalten und statt dessen eine Messelektronik an den Schallgeber oder an die daran angeordnete Signalelektrode anzuschalten.

Ab dem Zeitpunkt der Umschaltung erfolgt demnach ein gedämpftes Ausschwingen des Systems, welches durch die elektrische Signal-Wechselspannung repräsentiert wird, die an diesem Schallgeber bzw. an der mit diesem verbundenen Signalelektrode abgegriffen wird.

So besteht gemäß der Erfindung die Möglichkeit, mit der erwähnten Messelektronik die Signal-Wechseispannung zumindest zeitweise zu messen und aus deren Verlauf, insbesondere wie eingangs erwähnt, aus der so ermittelten Dämpfung ein Maß für die Verschmutzung der Raucheintrittsöffnungen zu errechnen.

Hierbei kann es in einer Ausführung bevorzugter Art vorgesehen sein, dass von der gemessenen Signal-Wechselspannung der Betrag aufeinander folgender Spannungsextrema gemessen wird. Hierbei kann es sich sowohl um Spannungsmaxima, also auch Spannungsminima handeln.

So reicht es erfindungsgemäß zur Bestimmung einer Dämpfung aus, lediglich den Betrag der Spannungsextremwerte der elektrischen Signal-Wechselspannung zu bestimmen. Der zwischen den Extremwerten liegende Spannungsverlauf kann ausser Acht gelassen werden. Es ist somit nicht zwingend nötig, einen hochaufgelösten zeitlichen Verlauf der Signal-Wechselspannung zu erfassen und zu speichern, sondern es kann vielmehr ein Maß für die Dämpfung aus wenigen diskreten Messpunkten erfolgen, die bevorzugterweise in der Position der jeweiligen Extrema, insbesondere der Maxima und/oder der Minima der Signal-Wechselspannung erfasst werden.

Aufgrund der Erfassung des Betrages von aufeinanderfolgenden Spannungsmaxima oder -minima, insbesondere von wenigstens zwei aufeinanderfolgenden Spannungsmaxima oder -minima, kann somit auf die Dämpfung des Systems zurückgeschlossen werden durch eine rechentechnische Auswertung dieser Maxima bzw. Minima. Beispielsweise kann eine Hüllkurve ermittelt werden, welche die Dämpfung des Systems repräsentiert.

In einer bevorzugten Ausführung kann es hier vorgesehen sein, dass aus der Spannungsdifferenz wenigstens zweier aufeinanderfolgender Spannungsmaxima oder -minima eine Dämpfung ermittelt wird. Hierbei kann die so ermittelte Dämpfung beispielsweise mit einem Vergleichswert verglichen werden, so dass durch diesen Vergleich ein objektives Maß für die Verschmutzung der Raucheintrittsöffnungen gegeben ist. Ein solcher Vergleichswert kann dabei z. B. als ein Dämpfungsminimum verstanden werden.

In einer anderen Ausführungsform kann es auch vorgesehen sein, dass schon alleine aus dem Betrag der Spannung eines oder mehrerer Signal-Wechselspannungsmaxima oder -minima, insbesondere ebenso nach einem Vergleich mit wenigstens einem Vergleichswert, ein Maß für die Verschmutzung der Raucheintrittsöffnungen gebildet wird. So kann es beispielsweise in einer Ausführung vorgesehen sein, dass nach einer pulsförmigen Anregung der Betrag der Spannung eines n-ten Wechelspannungsmaximum oder -minimum erfasst wird. Der Betrag der gemessenen Spannung kann somit verglichen werden mit dem Betrag der Spannung desselben n-ten Spannungsmaximums bzw. - minimums, der z.B. vorgelegen hat als der Rauchmelder ursprünglich installiert oder einer Zwischenwartung unterzogen wurde.

Dabei ist davon auszugehen, dass bei der Wartung, Erstinstallation oder sonstigen fest vorgegebenen definierten Situationen keine Verschmutzung gegeben ist und das so ermittelte Wechselspannungs-Maximum bzw. -minimum einen maximal bzw. minimal erreichbaren Wert darstellt. Hierbei kann es erfindungsgemäß vorgesehen sein, eine solche Messung nicht nur bezüglich eines WechselSpannungsmaximums oder -minimums durchzuführen, sondern in der Abklingkurve gegebenenfalls auch zu mehreren.

Der wenigstens eine Vergleichswert, der bei den genannten Ausführungen genutzt werden kann, kann z.B. durch Messung dieses Wertes nach einer Erstinstallation oder einer Wartung ermittelt und für spätere Vergleiche gespeichert werden.

In einer besonders bevorzugten Ausführungsform ist es vorgesehen, dass zur Messung des Betrags von aufeinanderfolgenden Spannungsmaxima oder - minima eine Messelektronik an einen periodischen Verlauf der Signal-Wechselspannung synchronisiert wird. Es besteht so die Möglichkeit, mit der synchronisierten Messelektronik die Spannung immer bevorzugt exakt im Maximum oder Minimum der periodisch aufgrund der Dämpfung abklingenden Signal-Wechselspannung zu messen.

Erfindungsgemäß kann es dabei vorgesehen sein, dass zur Realisierung einer solchen Synchronisierung die Messelektronik einen Komparator aufweist zur Feststellung der Periodendauer der Signal-Wechselspannung durch Messung der zeitlichen Abstände, an denen die Signal-Wechselspannung den zeitlichen Mittelwert erreicht.

Das festgestellte Intervall ist die Periodendauer der Signal-Wechselspannung. Daraus kann die Lage der weiteren Extremwerte ermittelt werden also der Maxima oder der Minima. So liegt diesem Messprinzip die Überlegung zugrunde, dass nach einem festgestellten Nulldurchgang ein Spannungsmaximum oder -minimum nach der Zeit folgt, die durch die Hälfte des zeitlichen Abstandes zwischen zwei Nulldurchgängen gegeben ist. So besteht hier die Möglichkeit, aufgrund der festgestellten Periodendauer und einem festgestellten Nulldurchgang ein Triggersignal oder ein Sample/Hold-Signal mit der Messelektronik zu erzeugen, um diese oder einen AD-Wandler zu veranlassen, im Zeitpunkt des Auftretens dieses Signals den instantan gemessenen Spannungswert zu speichern, zu digitalisieren und für die nachfolgende Auswertung zur Verfügung zu stellen.

So kann beispielsweise durch Mehrfachtriggerung nacheinander im Abstand der Periodendauer das jeweilige Spannungsmaximum oder -minimum, welches zum Zeitpunkt der Triggerung bzw. des Sample- und Hold-Signals vorliegt, erfasst werden und so mehrere zeitlich aufeinanderfolgende Beträge der Spannungsmaxima bzw. -minima gespeichert werden, um aus diesen Werten ein Maß für die Verschmutzung, insbesondere anhand der so ermittelbaren Dämpfung zu errechnen.

Erfindungsgemäß kann dafür die Messelektronik einen Analog-Digital-Wandler aufweisen, mittels dem die Spannungsbeträge der aufeinander folgenden Spannungsmaxima bzw. der -minima der Signal-Wechselspannung erfassbar bzw. digitalisierbar sind in Abhängigkeit dieses eingangs genannten Trigger- bzw. Sample- und Hold-Signals, welches entweder direkt oder nach einer Wandlung aus dem Signal des vorgenannten Komparators hergeleitet ist.

Es kann erfindungsgemäß weiterhin vorgesehen sein, dass das beschriebene Verfahren periodisch durchgeführt wird und der Rauchmelder ein Signal gibt, wenn das ermittelte Maß für die Verschmutzung eine vorgegebene Verschmutzungsgrenze erreicht bzw. überschreitet. Dieses Überschreiten kann wie eingangs genannt durch den Vergleich mit einem gespeicherten Vergleichswert und die Abweichung von diesem festgestellt werden.

In einer weiteren Ausführung kann es auch vorgesehen sein, dass ein Vergleichswert aus wenigstens zwei aufeinander folgenden gemessenen Maxima bzw. Minima verwendet wird. Hier kann dieser Vergleichswert ebenso wie der Messwert, mit dem verglichen wird, gegeben sein als der Logarithmus des Quotienten aus zwei aufeinander folgenden Maxima bzw. Minima.

Ein Ausführungsbeispiel der Erfindung ist in den nachfolgenden Figuren erläutert.

Es zeigen:
- Figur 1: den schematischen konstruktiven Aufbau eines erfindungsgemäßen Rauchmelders
- Figur 2: eine elektronische Schaltung, die in einem erfindungsgemäßen Rauchmelder zur Durchführung des Messprinzips realisiert ist.

- Figur 3: einen typischen Spannungsverlauf der Signal-Wechselspannung eines erfindungsgemäßen Rauchmelders, der mit dem erfindungsgemäßen Verfahren ermittelt wurde.

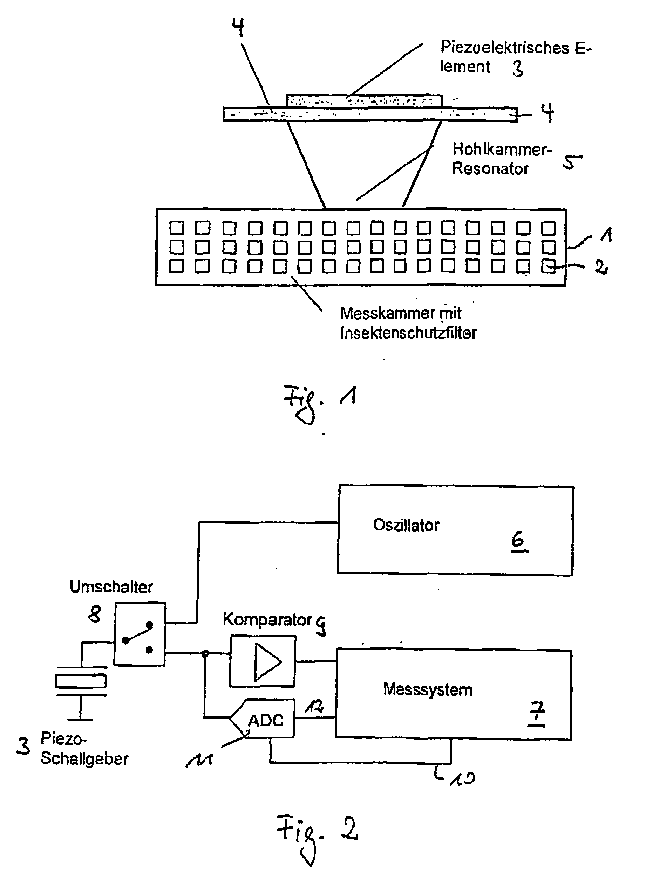

Die Figur 1 zeigt in einer Übersichtsdarstellung schematisch und stark vereinfacht einen erfindungsgemäßen Rauchmelder. Erkennbar ist hier eine Messkammer 1, die durch eine Vielzahl von Raucheintrittsöffnungen 2 zur Umgebung hin offen ist. Diese Öffnungen 2 dienen dazu, dass mit Rauch versehene Raum- oder Gebäudeluft in die Messkammer 1 eindringen kann, verhindern aber gleichzeitig das Eindringen von grobem Schmutz oder von Kleintieren, wie Insekten oder Spinnentieren.

Erkennbar ist hier weiterhin, dass ein piezoelektrisches Element 3 vorgesehen ist, welches sich auf einer Platte 4 befindet, die als Schallgeber arbeitet. Durch eine elektrische Ansteuerung des piezoelektrischen Elementes 3 mit einer insbesondere gepulsten Wechselspannung und dadurch hervorgerufene Schwingungen dieses Elementes wird die Platte 4, die als Schallgeber arbeitet, in Schwingungen versetzt, so dass Schallwellen durch den an der Platte 4 angekoppelten Hohlkammerresonator 5 in die Messkammer 1 eingeleitet werden und durch die Öffnungen 2 die Kammer verlassen.

Das erfindungsgemäße Verfahren kann hier in bevorzugter Weise mit der in der Figur 2 dargestellten elektrischen Schaltung realisiert werden. Hier ist erkennbar, dass die Gesamtanordnung dieser elektrischen Schaltung einen Oszillator 6 umfasst, ebenso wie eine Elektronik aus einem elektrischen Messsystem 7, einen Komparator 9 und einen Analog / Digital - Wandler 12. Oszillator 6 und Elektronik 7,9,11, insbesondere der Komparator 9 und der AD-Wandler 11 hiervon, können über einen Umschalter 8 wahlweise an den Piezoschallgeber 3 angeschaltet werden.

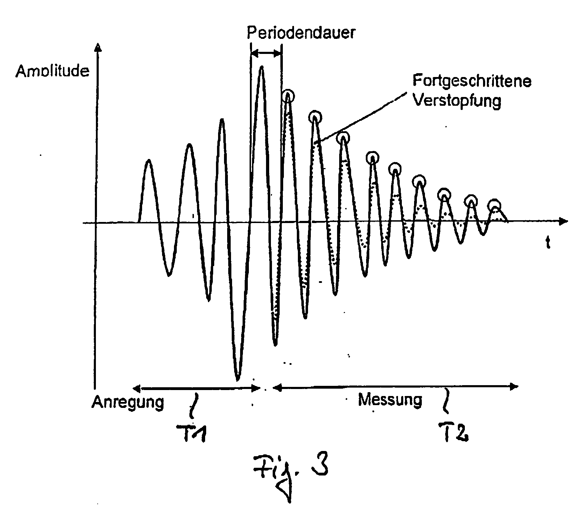

In der hier dargestellten Schaltstellung des Umschalters 8 wird die WechselSpannung, wie sie durch den Oszillator 6 erzeugt wird, an den Piezoschallgeber 3 angeschaltet, so dass dieser die Schallgeberplatte 4 in Schwingungen versetzt. Nach einem Abschalten, d.h, hier das Umschalten des Umschalters 8 in die untere Schaltposition, wird die anregende Spannung des Oszillators von dem Piezoschallgeber abgeschaltet, wodurch die Schwingung des Piezoschallgebers 3 bzw. der Platte 4 gedämpft ausschwingt. Der Piezoschallgeber 3 bzw. ein daran angeordneter Sensor dient ab diesem Augenblick als Signalspannungserzeuger mit einer gedämpften Schwingung, wobei die gemessene Signalspannung gemäß der hier dargestellten elektronischen Schaltung an einen Komparator 9 angeschaltet wird, der als Teil des gesamten Messsystems zu verstehen ist und mit dem die Nulldurchgänge der Signalspannung, wie sie beispielsweise der Figur 3 zu entnehmen ist, ermittelt werden können.
Das Messsystem 7 bildet aus dem Signal des Komparators 9 ein Signal, insbesondere ein Trigger- bzw. Sample- und Holdsignal, welches über die Leitung 10 an einen Analog-Digital-Wandler 11 angelegt werden kann, um diesen zu veranlassen, bei Erhalt des Signals über die Leitung 10 den aktuellen Spannungswert zu messen und zu digitalisieren.

Der gemessene und digitalisierte Spannungswert kann sodann über die Leitung 12 vom Analog-Digital-Wandler 11 ebenso an das Messsystem 7 zur Verfügung gestellt werden, um mit dem Messsystem 7 den Betrag der Spannung des gerade ausgemessenen Spannungsmaximums oder auch -minimums einer weiteren Berechnung zugrunde zu legen, um hier insbesondere durch Berechnung der Dämpfung auf die Verschmutzung zu schließen. Mit dem besagten Messsystem kann so durch wiederholtes und mit dem Signalverlauf durch den Komparator 9 synchronisiertes Messen der Amplitude der Signalwechselspannung die Dämpfung ermittelt werden und dies kann vorzugsweise ausgedrückt werden durch das logarithmische Dekrement, also das logarithmische Verhältnis zweier aufeinander folgender Amplituden von Maxima oder auch Minima. Dieses so ermittelte Maß ist damit unmittelbar ein Maß für die Veränderung der Verschmutzung der Raucheintrittsöffnungen der Messkammer und kann mit einem auf dieselbe Weise erhaltenen Vergleichwert eines oder exakt dieses Rauchmelders im sauberen Zustand verglichen werden.

Wie eingangs erwähnt, kann hier ein Initialwert als Vergleichswert und gegebenenfalls auch weitere Ausgangswerte mindestens bei einer Installation, gegebenenfalls aber auch bei späteren Kontrollen eines erfindungsgemäßen Rauchwarnmelders bestimmt werden, um so unterschiedlichen akustischen Umgebungsbedingungen sowie der Ankopplung des Gehäuses an den Montageort Rechnung zu tragen.

Die Figur 3 zeigt einen typischen Spannungsverlauf, wie er mit dem erfindungsgemäßen Verfahren erzeugt und ausgewertet werden kann. Hier ist insbesondere erkennbar, dass eine anfängliche Zeit T1 verwendet wird, um den Piezosignalgeber 3 anzuregen. Nach der Anregungszeit T1, nach welcher der zuvor genannte Schalter 8 umgelegt wird, folgt eine Messzeit T2, bei der erkennbar ist, dass der Signalverlauf der elektrischen Signal-Wechselspannung mit der Zeit aufgrund der vorliegenden Dämpfung im System abnimmt.

Durch die eingangs genannte elektronische Messschaltung, insbesondere den Komparatoraufbau 9, kann zum einen die Feststellung der Nulldurchgänge erfolgen und aufgrund der Kenntnis über die Nulldurchgänge zurückgeschlossen werden auf die Periodendauer. Um eine halbe Periodendauer verschoben, kann somit nach jedem festgestellten Nulldurchgang exakt ein Spannungsmaximum bzw. Minimum durch die zuvor benannte Sample- und Holdspannungswandlung der gemessenen Analogwerte in Digitalwerte der Betrag des jeweils betrachteten Spannungsmaximums gemessen werden. Hier ist beispielhaft die Vermessung der Maxima dargestellt.

Hier ist erkennbar, dass mit der durchgezogenen Linie eines Spannungsverlaufs eine ursprüngliche initiale Messung im Vergleich zu einer späteren Messung dargestellt ist, bei der mit dem gepunkteten Verlauf erkennbar ist, dass die Dämpfung deutlich gegenüber der Ursprungsmessung zugenommen hat.

Es können somit beispielsweise verschiedene Auswertungsarten der eingangs genannten Art verwendet werden, um auf die Verschmutzung zurückzuschließen. Bevorzugt wird hierbei wie zuvor genannt, das logarithmische Dekrement zwischen zwei aufeinander folgenden Spannungsmaxima oder -minima eingesetzt.

Im Rahmen der Erfindung werden dabei auch jegliche andere Rechenverfahren als erfindungsgemäß betrachtet, die geeignet sind, um zumindest aus den gemessenen Werten von einigen (wenigstens zwei), aufeinander folgenden Spannungsmaxima bzw -minima, gegebenenfalls auch aus höher aufgelösten Spannungsmessungen ein Maß für die Dämpfung aus dem Signal-Wechselspannungsverlauf zu ermitteln.

Bei der Verwendung des erfindungsgemäßen Verfahrens spielt dabei auch eine eventuelle Verschiebung der Resonanzfrequenz nur eine untergeordnete Rolle, da durch die eingesetzte Komparatorschaltung die jeweilige Periodendauer, wie sie bei einer bestimmten Verschmutzungsbedingung vorliegt, unmittelbar und genau ermittelt wird. Das hier beschriebene Messprinzip ist daher unanfällig gegen eventuelle Verschiebungen dieser Resonanzfrequenz und betrachtet lediglich die Auswirkungen hinsichtlich der Dämpfung des Systems.

Dabei kann selbstverständlich das System in einer Resonanzbedingung betrieben werden, dies ist jedoch nicht zwingend nötig. Der Vorteil des Betriebs in einer Resonanzbedingung kann hierbei darin gesehen werden, dass höhere Absolutbeträge der gemessenen Spannungsmaxima erzielt werden können, wohingegen außerhalb einer Resonanzbedingung grundsätzlich die Dämpfungen größer sind. Es können daher Vorteile sowohl bei der Messung innerhalb einer Resonanzbedingung als auch außerhalb der Resonanzbedingung erkannt werden.

Bezüglich sämtlicher Ausführungen ist festzustellen, dass die in Verbindung mit einer Ausführung genannten technischen Merkmale nicht nur bei der spezifischen Ausführung eingesetzt werden können oder eingesetzt sind, sondern auch bei den jeweils anderen Ausführungen. Sämtliche offenbarten technischen Merkmale dieser Erfindungsbeschreibung sind als erfindungswesentlich einzustufen und beliebig miteinander kombinierbar oder in Alleinstellung einsetzbar. Dabei wird in der gesamten Offenbarung unter der Erwähnung, dass ein Merkmal vorgesehen sein kann oder ein Verfahrenschritt durchgeführt werden kann auch eine Ausführung der Erfindung verstanden, in der das betreffende Merkmal vorgesehen ist bzw. ein betreffender Verfahrensschritt durchgeführt wird.

## Patentansprüche

1. Verfahren zur Feststellung der Verschmutzung der Raucheintrittsöffnungen eines Rauchwarnmelders mit einem elektroakustischen Schallgeber, **dadurch gekennzeichnet, dass** der Schallgeber (3,4) in einem ersten Schritt zur Erzeugung von Schall, insbesondere durch transiente elektrische Anregung, erregt wird und in einem zweiten nachfolgenden Schritt eine vom abklingenden Schall abhängige elektrische Signal-Wechselspannung zumindest zeitweise gemessen wird, aus deren Verlauf ein Maß für die Verschmutzung der Raucheintrittsöffnungen (2) errechnet wird.

2. Verfahren nach Anspruch 1, **dadurch gekennzeichnet, dass** die Signal-Wechselspannung direkt am Schallgeber (3,4) oder an einer mit diesem verbundenen Signalelektrode abgegriffen wird.

3. Verfahren nach einem der vorherigen Ansprüche, **dadurch gekennzeichnet, dass** von der Signal-Wechselspannung der Betrag aufeinanderfolgender Spannungsextrema, insbesondere aufeinanderfolgender Spannungsmaxima oder aufeinanderfolgender Spannungsminima, gemessen wird.

4. Verfahren nach Anspruch 3, **dadurch gekennzeichnet, dass** aus der Spannungsdifferenz wenigstens zweier aufeinanderfolgender Spannungsextrema, insbesondere aufeinanderfolgender Spannungsmaxima oder aufeinanderfolgender Spannungsminima, eine Dämpfung ermittelt wird, die, insbesondere nach einem Vergleich mit wenigstens einem Vergleichswert ein Maß für die Verschmutzung der Raucheintrittsöffnungen (2) bildet.

5. Verfahren nach Anspruch 3, **dadurch gekennzeichnet, dass** aus dem Betrag der Spannung eines oder mehrerer Signal-Wechselspannungsextrema, insbesondere nach einem Vergleich mit wenigstens einem Vergleichswert, ein Maß für die Verschmutzung der Raucheintrittsöffnungen gebildet wird.

6. Verfahren nach einem der vorherigen Ansprüche 4 oder 5, **dadurch gekennzeichnet, dass** zur Bestimmung wenigstens eines Vergleichswertes die vom abklingenden Schall abhängige Signal-Wechselspannung, insbesondere die Beträge von deren aufeinanderfolgenden Spannungsextrema, insbesondere aufeinanderfolgender Spannungsmaxima oder aufeinanderfolgender Spannungsminima, wenigstens nach einer Erstinstallation eines unverschmutzten Rauchmelders und/oder bei Kontrollmessungen zumindest zeitweise gemessen wird

7. Verfahren nach einem der vorherigen Ansprüche 3 bis 6, **dadurch gekennzeichnet, dass** zur Messung des Betrages aufeinanderfolgender Spannungsextrema, insbesondere aufeinanderfolgender Spannungsmaxima oder aufeinanderfolgender Spannungsminima, eine Messelektronik (7,9,11) an den periodischen Verlauf der Signal-Wechselspannung synchonisiert wird.

8. Verfahren nach Anspruch 7, **dadurch gekennzeichnet, dass** zur Synchronisation die Nulldurchgänge der Signal-Wechselspannung, insbesondere mit einer Komparatorschaltung (9), erfasst werden und aus den Nulldurchgängen die Periodendauer der Signal-Wechselspannung und aus beiden die zeitliche Lage der Spannungsextrema ermittelt wird, insbesondere zur Erzeugung eines Trigger- und/oder Sample/Hold-Signales der Messelektronik (7,9,11).

9. Verfahren nach einem der vorherigen Ansprüche, **dadurch gekennzeichnet, dass** es periodisch durchgeführt wird und der Rauchmelder ein Signal gibt, wenn das ermittelte Maß für die Verschmutzung eine vorgegebene Verschmutzungsgrenze erreicht oder überschreitet.

10. Verfahrend nach einem der Ansprüche 3-9, **dadurch gekennzeichnet, dass** ein Meßwert und oder Vergleichswert aus wenigstens zwei aufeinanderfolgenden Extrema, insbesondere aufeinanderfolgenden Spannungsmaxima oder aufeinanderfolgenden Spannungsminima, gebildet wird.

11. Verfahren nach Anspruch 10, **dadurch gekennzeichnet, dass** der Meßwert oder Vergleichswert dem Logarithmus des Quotienten aus zwei aufeinanderfolgenden Extrema, insbesondere aufeinanderfolgenden Spannungsmaxima oder aufeinanderfolgenden Spannungsminima, entspricht.

12. Rauchmelder mit einer Messkammer, die durch Raucheintrittsöffnungen gegen die äußere Umgebung abgegrenzt ist, wobei an die Messkammer ein Schallgeber, insbesondere über einen Hohlkammerresonator angekoppelt ist und mit einer Elektronik zur Überprüfung der Verschmutzung der Raucheintrittsöffnungen, **dadurch gekennzeichnet, dass** die Elektronik (3,8,7,8,10,11,12) einen Schalter (8) umfasst, mittels dem wahlweise ein Oszillator (6) oder eine Messelektronik (7,9,10,11,12) an den Schallgeber (3,) oder eine daran angeordnete Signalelektrode anschaltbar ist, wobei die Messelektronik (7,9,10,11,12) eingerichtet ist, die Beträge aufeinanderfolgender Spannungsextrema, insbesondere aufeinanderfolgender Spannungsmaxima oder aufeinanderfolgender Spannungsminima, einer Signal-Wechselspannung zu erfassen, die am Schallgeber (3) oder dessen Signalelektrode nach einer vom Oszillator (6) erzeugten und mit dem Schalter (8) abgeschalteten Anregungs-Wechselspannung anliegt und zeitlich abklingt.

13. Rauchmelder nach Anspruch 12, **dadurch gekennzeichnet, dass** die Messelektronik (7,9,10,11,12) einen Komparator (9) aufweist zur Feststellung der Periodendauer der Signalwechselspannung durch Messung von deren Nulldurchgängen.

14. Rauchmelder nach Anspruch 13, **dadurch gekennzeichnet, dass** die Messelektronik (7,9,10,11,12) einen Analog-Digital-Wandler (11) aufweist, mittels dem die Spannungsbeträge der aufeinanderfolgenden Spannungsextrema, insbesondere aufeinanderfolgender Spannungsmaxima oder aufeinanderfolgender Spannungsminima, der Signal-Wechselspannung erfassbar sind in Abhängigkeit von einem aus dem Signal des Komparators (9) hergeleiteten Trigger-Signals.

## Claims

1. Method for determining the contamination of the smoke inlet openings of a smoke alarm with an electroacoustic sound generator, **characterized in that**, in a first step, the sound generator (3, 4) is excited to generate sound, in particular by means of transient electrical excitation, and, in a second, subsequent step, an electrical signal AC voltage which is dependent on the decaying sound is at least temporarily measured, from the profile of which a measure of the contamination of the smoke inlet openings (2) is calculated.

2. Method according to Claim 1, **characterized in that** the signal AC voltage is directly tapped off at the sound generator (3, 4) or at a signal electrode connected to the latter.

3. Method according to one of the preceding claims, **characterized in that** the magnitude of successive voltage extremes, in particular successive voltage maxima or successive voltage minima, of the signal AC voltage is measured.

4. Method according to Claim 3, **characterized in that** the voltage difference between at least two successive voltage extremes, in particular successive voltage maxima or successive voltage minima, is used to determine attenuation which forms a measure of the contamination of the smoke inlet openings (2), in particular after a comparison with at least one comparison value.

5. Method according to Claim 3, **characterized in that** the magnitude of the voltage of one or more signal AC voltage extremes is used to form a measure of the contamination of the smoke inlet openings, in particular after a comparison with at least one comparison value.

6. Method according to either of the preceding Claims 4 and 5, **characterized in that**, in order to determine at least one comparison value, the signal AC voltage dependent on the decaying sound, in particular the magnitudes of its successive voltage extremes, in particular successive voltage maxima or successive voltage minima, is at least temporarily measured at least after initial installation of an uncontaminated smoke alarm and/or during control measurements.

7. Method according to one of the preceding Claims 3 to 6, **characterized in that**, in order to measure the magnitude of successive voltage extremes, in particular successive voltage maxima or successive voltage minima, measuring electronics (7, 9, 11) are synchronized with the periodic profile of the signal AC voltage.

8. Method according to Claim 7, **characterized in that**, for the purpose of synchronization, the zero crossings of the signal AC voltage are detected, in particular using a comparator circuit (9), and the period duration of the signal AC voltage is determined from the zero crossings and the temporal position of the voltage extremes is determined from both, in particular for the purpose of generating a trigger signal and/or a sample/hold signal of the measuring electronics (7, 9, 11).

9. Method according to one of the preceding claims, **characterized in that** it is periodically carried out, and the smoke alarm provides a signal if the determined measure of the contamination reaches or exceeds a predefined contamination limit.

10. Method according to one of Claims 3-9, **characterized in that** a measured value and/or a comparison value is/are formed from at least two successive extremes, in particular successive voltage maxima or successive voltage minima.

11. Method according to Claim 10, **characterized in that** the measured value or comparison value corresponds to the logarithm of the quotient of two successive extremes, in particular successive voltage maxima or successive voltage minima.

12. Smoke alarm having a measuring chamber which is delimited from the external environment by smoke inlet openings, a sound generator being coupled to the measuring chamber, in particular via a hollow chamber resonator, and having electronics for checking the contamination of the smoke inlet openings, **characterized in that** the electronics (3, 6, 7, 8, 10, 11, 12) comprise a switch (8) which can be used to optionally connect an oscillator (6) or measuring electronics (7, 9, 10, 11, 12) to the sound generator (3) or a signal electrode arranged on the latter, the measuring electronics (7, 9, 10, 11, 12) being set up to detect the magnitudes of successive voltage extremes, in particular successive voltage maxima or successive voltage minima, of a signal AC voltage which is applied to the sound generator (3) or its signal electrode according to an excitation AC voltage generated by the oscillator (6) and switched off using the switch (8) and decays over time.

13. Smoke alarm according to Claim 12, **characterized in that** the measuring electronics (7, 9, 10, 11, 12) have a comparator (9) for determining the period duration of the signal AC voltage by measuring the zero crossings of the latter.

14. Smoke alarm according to Claim 13, **characterized in that** the measuring electronics (7, 9, 10, 11, 12) have an analogue/digital converter (11) which can be used to detect the voltage magnitudes of the successive voltage extremes, in particular successive voltage maxima or successive voltage minima, of the signal AC voltage on the basis of a trigger signal derived from the signal from the comparator (9).

## Revendications

1. Procédé pour déterminer l'encrassement des ouvertures d'entrée de la fumée d'un avertisseur de fumée muni d'un émetteur sonore électroacoustique, **caractérisé en ce que** l'émetteur sonore (3, 4), dans une première étape, est excité pour générer un son, notamment par une excitation électrique transitoire, et, dans une deuxième étape suivante, une tension alternative de signal électrique dépendante du son décroissant est mesurée au moins temporairement et une mesure de l'encrassement des ouvertures d'entrée de la fumée (2) est calculée à partir de son tracé.

2. Procédé selon la revendication 1, **caractérisé en ce que** la tension alternative de signal est prélevée directement sur l'émetteur sonore (3, 4) ou sur une électrode de signal reliée avec celui-ci.

3. Procédé selon l'une des revendications précédentes, **caractérisé en ce que** la valeur d'extrêmes de tension successifs de la tension alternative de signal, notamment de maximums de tension successifs ou de minimums de tension successifs, est mesurée.

4. Procédé selon la revendication 3, **caractérisé en ce qu'**une atténuation est déterminée à partir de la différence de tension entre au moins deux extrêmes de tension successifs, notamment des maximums de tension successifs ou des minimums de tension successifs, laquelle forme une mesure de l'encrassement des ouvertures d'entrée de la fumée (2), notamment après une comparaison avec au moins une valeur comparative.

5. Procédé selon la revendication 3, **caractérisé en ce qu'**une mesure de l'encrassement des ouvertures d'entrée de la fumée est formée à partir de la valeur de la tension d'un ou plusieurs extrêmes de tension alternative, notamment après une comparaison avec au moins une valeur comparative.

6. Procédé selon l'une des revendications précédentes 4 ou 5, **caractérisé en ce que** pour déterminer au moins une valeur comparative, la tension alternative de signal dépendante du son décroissant, notamment les valeurs de ses extrêmes de tension successifs, notamment des maximums de tension successifs ou des minimums de tension successifs, est mesurée au moins après une première installation d'un avertisseur de fumée non encrassé et/ou au moins temporairement lors des mesures de contrôle.

7. Procédé selon l'une des revendications précédentes 3 à 6, **caractérisé en ce que** pour mesurer la valeur des extrêmes de tension successifs, notamment des maximums de tension successifs ou des minimums de tension successifs, une électronique de mesure (7, 9, 11) est synchronisée sur le tracé périodique de la tension alternative de signal.

8. Procédé selon la revendication 7, **caractérisé en ce que** les passages par zéro de la tension alternative de signal sont détectés, notamment avec un circuit comparateur (9), en vue de la synchronisation, et la durée de la période de la tension alternative de signal est déterminée à partir des passages par zéro et la position dans le temps des extrêmes de tension est déterminée à partir des deux, notamment en vue de générer un signal de déclenchement et/ou d'échantillonnage-blocage de l'électronique de mesure (7, 9, 11).

9. Procédé selon l'une des revendications précédentes, **caractérisé en ce qu'**il est exécuté périodiquement et l'avertisseur de fumée délivre un signal lorsque la mesure déterminée pour l'encrassement atteint ou dépasse une limite d'encrassement prédéfinie.

10. Procédé selon l'une des revendications précédentes 3 à 9, **caractérisé en ce qu'**une valeur mesurée ou une valeur comparative est formée à partir d'au moins deux extrêmes successifs, notamment des maximums de tension successifs ou des minimums de tension successifs.

11. Procédé selon la revendication 10, **caractérisé en ce que** la valeur mesurée ou la valeur comparative correspond au logarithme des quotients de deux extrêmes successifs, notamment des maximums de tension successifs ou des minimums de tension successifs.

12. Avertisseur de fumée comprenant une chambre de mesure, laquelle est délimitée de l'environnement externe par des ouvertures d'entrée de la fumée, un émetteur sonore étant accouplé à la chambre de mesure, notamment par le biais d'un résonateur à chambre creuse, et comprenant une électronique pour contrôler l'encrassement des ouvertures d'entrée de la fumée, **caractérisé en ce que** l'électronique (3, 6, 7, 8, 10, 11, 12) comprend un commutateur (8) au moyen duquel un oscillateur (6) ou une électronique de mesure (7, 9, 10, 11, 12), au choix, peut être connecté à l'émetteur sonore (3) ou à une électrode de signal disposée sur celui-ci, l'électronique de mesure (7, 9, 10, 11, 12) étant configurée pour détecter les valeurs des extrêmes de tension successifs, notamment de maximums de tension successifs ou de minimums de tension successifs, d'une tension alternative de signal, laquelle est appliquée à l'émetteur sonore (3) ou à son électrode de signal d'après une tension alternative d'excitation générée par l'oscillateur (6) et déconnectée avec le commutateur (8) et décroît dans le temps.

13. Avertisseur de fumée selon la revendication 12, **caractérisé en ce que** l'électronique de mesure (7, 9, 10, 11, 12) présente un comparateur (9) pour déterminer la durée de la période de la tension alternative de signal en mesurant ses passages par zéro.

14. Avertisseur de fumée selon la revendication 13, **caractérisé en ce que** l'électronique de mesure (7, 9, 10, 11, 12) présente un convertisseur analogique/numérique (11) au moyen duquel peuvent être détectées les valeurs de la tension des extrêmes de tension successifs, notamment de maximums de tension successifs ou de minimums de tension successifs, de la tension de signal alternative en fonction d'un signal de déclenchement dérivé du signal du comparateur (9).
